# EUROPEAN PATENT SPECIFICATION

(11) **EP 2 865 377 B1**
(45) Date of publication and mention of the grant of the patent: **14.10.2020**
(21) Application number: 13807330.9
(22) Date of filing: 18.06.2013
(51) Int. Cl.: A61K 31/196, A61K 9/70, A61K 47/08, A61K 47/32, A61P 29/00, A61K 47/34, A61K 47/46

(54) **PERCUTANEOUS ABSORPTION PROMOTER AND SKIN PATCH COMPRISING SAME**
PERKUTANER ABSORPTIONSPROMOTOR UND HAUTPFLASTER DAMIT
AGENT FAVORISANT L'ABSORPTION PERCUTANÉE ET PATCH CUTANÉ EN CONTENANT

(30) Priority: 20.06.2012 JP 2012138805
(43) Date of publication of application: 29.04.2015
(73) Proprietor: Hisamitsu Pharmaceutical Co., Inc., Tosu-shi, Saga 841-0017 (JP)
(72) Inventor: YOSHINAGA Takaaki, Tosu-shi Saga 841-0017 (JP); SATO Masahiro, Tosu-shi Saga 841-0017 (JP); OISHI Akira, Tosu-shi Saga 841-0017 (JP); KOSE Yasuhisa, Tosu-shi Saga 841-0017 (JP)
(74) Representative: Grünecker Patent- und Rechtsanwälte PartG mbB
(86) International application number: PCT/JP2013/066663
(87) International publication number: WO 2013/191158

(56) References cited:
- EP-A1- 0 698 393
- EP-A1- 1 400 240
- WO-A1-2004/071499
- WO-A1-2008/133272
- WO-A1-2011/158964
- JP-A- H10 182 450
- JP-A- 2006 045 158
- JP-A- 2006 160 707
- JP-A- 2010 030 909
- JP-A- 2011 121 980
- JP-B2- 2 852 816

## Description

### [Technical Field]

The present invention relates to a transdermal absorption promoting agent and a patch comprising it.

### [Background Art]

Although diclofenac is an excellent nonsteroidal analgesic anti-inflammatory agent, there is a problem that there are cases where it shows side effects such as gastrointestinal disorders when it is orally administered. For this reason, a variety of topical agents for external use whose effective ingredients are diclofenac have been developed and patches comprising diclofenac sodium have been marketed as drugs for topical analgesic application in Japan.

In addition, there are many cases where diclofenac comprised in those patches is used in the form of a pharmaceutically acceptable salt, particularly a sodium salt from the standpoints of improving its stability, suppressing reduction in physical properties (intensity, elasticity, durability, adhesiveness) of adhesive agent layers of the patches, decreasing irritation to the skin, etc. However, diclofenac sodium is scarcely soluble in ether but is relatively soluble in water; since its percutaneous absorbability is insufficient, a variety of measures have been taken.

For example, in Japanese Unexamined Patent Application No. Sho 61-280426 (Patent Literature 1), there is described that by using an organic acid such as citric acid in combination with diclofenac sodium to have them comprised in the pressure-sensitive adhesive material layer of an analgesic anti-inflammatory patch, the solubility and skin permeability of diclofenac sodium in the patch are improved. Also, in Japanese Unexamined Patent Application No. Sho 62-181226 (Patent Literature 2), there is described that by using a glycol in addition to an organic acid such as citric acid in combination with diclofenac sodium to have them comprised in the base of an analgesic anti-inflammatory patch, the solubility and skin permeability of diclofenac sodium in the patch are improved. Further, in Japanese Unexamined Patent Application No. 2002-338462 (Patent Literature 3), there is described that by combining and compounding diclofenac sodium, an organic acid such as citric acid, pyrrolidone or a derivative thereof, and an aliphatic acid ester of polyvalent alcohol in the base of an adhesive poultice, diclofenac sodium is stably solubilized in the base to improve the releasability from the poultice and the percutaneous absorbability. Additionally, in Japanese Unexamined Patent Application No. Hei 11-322595 (Patent Literature 4), there is described that by providing a drug reservoir layer which comprises diclofenac sodium and an organic acid such as citric acid at a predetermined weight ratio, a diffusion control membrane for diclofenac sodium, and a hydrophobic adhesive layer capable of being attached to the skin, the excellent skin permeability of diclofenac sodium can be displayed and the antipyretic, analgesic anti-inflammatory action can be exerted systemically.

Also, drugs in free forms are normally superior in percutaneous absorbability to their salts in patches. Therefore, as is disclosed in Patent Literature 1, techniques of simultaneously compounding diclofenac sodium salt and an organic acid such as citric acid, and desalting during production or formulation have been investigated with respect to diclofenac-containing preparations as well.

### [Citation List]

### [Patent Literature]

[PTL 1] Japanese Unexamined Patent Application No. Sho 61-280426
[PTL 2] Japanese Unexamined Patent Application No. Sho 62-181226
[PTL 3] Japanese Unexamined Patent Application No. 2002-338462
[PTL 4] Japanese Unexamined Patent Application Hei 11-322595

EP 0 698 393 relates to a solubilizing agent or solubilizer for a pharmaceutically effective ingredient and an external preparation comprising the solubilizer. WO 2011/158964 discloses a transdermal absorption promoter and an external skin formulation comprising the transdermal absorption promoter. EP 1 400 240 relates to a percutaneous absorbable patch.

### [Summary of Invention]

### [Technical Problem]

As described above, the skin permeability of diclofenac sodium in patches will be improved by virtue of the inclusion of organic acids such as citric acid. However, as a result of the studies by the present inventors, it has become clear that by simultaneously compounding diclofenac sodium and citric acid in preparations, the stability of diclofenac sodium lowers in a time-dependent manner in the adhesive agent layers of diclofenac-containing patches, as will be shown in the Examples to be mentioned later. Therefore, since the time-dependent lowered stability of diclofenac sodium results in the analgesic anti-inflammatory patches as described in Patent Literatures 1-4, these analgesic anti-inflammatory patches are not sufficient from the standpoint that diclofenac sodium is comprised stably in the adhesive agent layers.

The present invention has been made in view of the above-described problems of the prior art, and an object of the present invention is to provide a patch which can comprise diclofenac sodium stably and at the same time which is excellent in the skin permeability of diclofenac.

### [Solution to Problem]

The present inventors have conducted earnest studies to achieve the above-described object, and consequently have revealed that l-menthyl glyceryl ether is excellent in the solubilizing power for diclofenac sodium among a large number of existing drug solubilizers. Moreover, they have found that by including l-menthyl glyceryl ether and a terpene-based resin into an adhesive agent layer of a patch, the time-dependent stability is excellent and further the skin permeability is improved for dichlofenac sodium and have completed the present invention.

Specifically, a transdermal absorption promoting agent of the present invention comprises l-menthyl glyceryl ether, and a terpene-based resin, wherein the terpene-based resin is a pinene polymer.

It is preferred that for the transdermal absorption promoting agent of the present invention, a mass ratio between the l-menthyl glyceryl ether and the terpene-based resin (mass of menthyl glyceryl ether : mass of terpene-based resin) is 1:0.1 to 1:20.

Further, the transdermal absorption promoting agent of the present invention preferably does not comprise any of citric acid, a glycol and a polyalkyleneglycol fatty acid ester.

Also, a patch of the present invention comprises a support layer and an adhesive agent layer, wherein the adhesive agent layer comprises diclofenac sodium and further comprises the transdermal absorption promoting agent of the present invention to promote percutaneous absorption of the diclofenac sodium.

Further, the adhesive agent layer according to the present invention preferably comprises 0.1 to 10 mass parts of the l-menthyl glyceryl ether and 1 to 40 mass parts of the terpene-based resin relative to 1 mass part of the diclofenac sodium.

Also, the adhesive agent layer according to the present invention preferably does not comprise any of citric acid, a glycol and a polyalkyleneglycol fatty acid ester.

Further, the terpene-based resin is a pinene polymer.

### [Advantageous Effects of Invention]

According to the present invention, it will be possible to provide a patch which can stably comprise diclofenac sodium and at the same time which is excellent in the skin permeability of diclofenac.

### [EMBODIMENTS FOR CARRYING OUT THE INVENTION]

Hereinafter, the present invention will be described in detail based on preferred embodiments thereof.

The transdermal absorption promoting agent of the present invention is a transdermal absorption promoting agent for promoting the percutaneous absorption of diclofenac sodium, comprising l-menthyl glyceryl ether, and a terpene-based resin, wherein the terpene-based resin is a pinene polymer.

Diclofenac sodium according to the present invention is a nonsteroidal type drug having analgesic, anti-inflammatory action and is a compound which is also referred to as sodium 2-[(2,6-dichlorophenyl)]amino]phenyl acetate.

l-Menthyl glyceryl ether according to the present invention is a compound which is also referred to as monomenthyl glyceryl ether or 3-1-menthoxypropane-1,2-diol. Note that l-menthyl glyceryl ether is known as a drug solubilizer. However, as will be shown in the Examples to be mentioned later, it has become clear for the first time in the present invention that by virtue of the use of 1-menthyl glyceryl ether, the time-dependent stability of diclofenac sodium enhances and further, the skin permeability of diclofenac sodium also improves.

Examples of the terpene-based resin are pinene polymers (a-pinene polymers, β-pinene polymers). More specifically, there are mentioned YS resins (YS RESIN PXN, YS RESIN PX1000, YS RESIN T0125, YS RESIN T0105), CLEARON P105, CLEARON M115, CLEARON K100 (all trade names; manufactured by YASUHARA CHEMICAL CO. LTD.), and TAMANOL 901 (trade name; Arakawa Chemical Industries, Ltd.). In addition, as for such terpene-based resins, one kind may be used alone, or two or more kinds may be used in combination. These terpene-based resins are pinene polymers, and preferably YS resins, from the standpoint that the sense of use is good because suitable attachability to the skin is easily obtainable and their odors are also less.

Note that the terpene-based resins are being normally used as tackifiers which are comprised in the adhesive agent layers of patches. However, as will be shown in the Examples to be mentioned later, it has become clear for the first time in the present invention that by virtue of the use of the terpene-based resins as opposed to other tackifiers such as hydrogenated rosin esters, the time-dependent stability of diclofenac sodium enhances, and further, the skin permeability of diclofenac also improves.

Also, as the resin comprised in the transdermal absorption promoting agent of the present invention, the terpene-based resin can be used singly, or a plurality of them can be used in combination. Between these, from the standpoint of less skin irritability, it is preferable to use a pinene polymer singly; and it is more preferable to use a YS resin singly.

In the transdermal absorption promoting agent of the present invention, a content of the l-menthyl glyceryl ether is preferably 0.1 to 20% by mass, and more preferably, 3 to 10% by mass relative to the total mass of the transdermal absorption promoting agent. If the content of l-menthyl glyceryl ether is less than the lower limit, the solubilizing power toward diclofenac sodium is insufficient and the skin permeability of diclofenac by a patch comprising this transdermal absorption promoting agent tends to lower, while the pain upon peeling off a patch tends to increase. On the other hand, if it exceeds the upper limit, diclofenac analog substances are likely to increase in the adhesive agent layer of the patch comprising this transdermal absorption promoting agent, while stickiness or bleed tends to be easily caused in the patch.

Also, a content of the terpene-based resin is preferably 1 to 40 % by mass, and more preferably, 13 to 20% by mass relative to the total mass of the transdermal absorption promoting agent. If the content of the terpene-based resin is less than the lower limit, the attachability of a patch comprising this transdermal absorption promoting agent to the skin tends to lower, while the coating aptness of an adhesive agent composition comprising this transdermal absorption promoting agent tends to deteriorate. On the other hand, if it exceeds the upper limit, the skin permeability of diclofenac by a patch comprising this transdermal absorption promoting agent tends to lower, the pain upon peeling off the patch tends to increase, and further stickiness or tongue-out (the adhesive agent layer sticking out from a support layer) tends to be easily caused in the patch comprising this transdermal absorption promoting agent.

Further, a mass ratio between the l-menthyl glyceryl ether and the terpene-based resin (mass of l-menthyl glyceryl ether : mass of terpene-based resin) is preferably 1:0.1 to 1:20, and more preferably, 1:2 to 1:10. If the ratio of the mass of the terpene-based resin to the mass of the l-menthyl glyceryl ether is less than the lower limit, the attachability to the skin tends to lower, while the coating aptness tends to deteriorate. On the other hand, if it exceeds the upper limit, the skin permeability of diclofenac by a patch comprising this transdermal absorption promoting agent tends to lower, while the pain upon peeling off the patch tends to increase.

The transdermal absorption promoting agent of the present invention can be produced by employing a known production method, as appropriate, without any particular limitation. The transdermal absorption promoting agent also may comprise other compounding agents as long as the excellent time-dependent stability of diclofenac sodium and the skin permeability of diclofenac are obtained by including the l-menthyl glyceryl ether and the terpene-based resin. However, the time-dependent stability of diclofenac sodium and/or the skin permeability of diclofenac to be improved by including the l-menthyl glyceryl ether and the terpene-based resin tends to easily lower in coexistence with citric acid, a glycol, or a polyalkyleneglycol fatty acid ester, each of which has been conventionally used as a transdermal absorption promoting agent of diclofenac. Accordingly, there is no need for the transdermal absorption promoting agent according to the present invention to even comprise citric acid, a glycol, or a polyalkyleneglycol fatty acid ester.

The citric acid according to the present invention is anhydrous citric acid, citric acid hydrate, a citrate (such as sodium citrate, potassium citrate, magnesium citrate, or calcium citrate).

Also, examples of the glycol according to the present invention include polypropylene glycol, ethylene glycol, diethylene glycol, triethylene glycol, butylene glycol, and hexylene glycol.

Further, examples of the polyalkylene glycol fatty acid ester according to the present invention include polyethylene glycol fatty acid esters such as polyethylene glycol monostearate, polyethylene glycol monolaurate, polyethylene glycol monoisostearate, polyethylene glycol monooleate, polyethylene glycol dilaurate, polyethylene glycol distearate, polyethylene glycol diisostearate, and polyethylene glycol dioleate, as well as propylene glycol fatty acid esters such as propylene glycol monostearate, propylene glycol monolaurate, and propylene glycol monooleate.

Next, a patch of the present invention will be described. The patch of the present invention comprises a support layer and an adhesive agent layer formed on a surface or surfaces (usually on one of the surfaces) of the support layer, wherein the adhesive agent layer comprises the diclofenac sodium and further comprises the transdermal absorption promoting agent of the present invention to promote percutaneous absorption of the diclofenac sodium.

In the patch of the present invention, a content of the diclofenac sodium is not particularly limited as long as it is an amount which exerts a pharmacological effect, and it can be adjusted, as appropriate, depending on the purpose of therapy. However, it is generally 0.1 to 20% by mass, and preferably, 0.5 to 10% by mass relative to the total mass of the adhesive agent layer. If the content is less than the lower limit, sufficient efficacy, i.e., analgesic anti-inflammatory effect, tends to unlikely be obtained. On the other hand, if it exceeds the upper limit, skin irritation tends to be easily caused.

In the patch of the present invention, a content of the l-menthyl glyceryl ether is preferably 0.1 to 20% by mass, and more preferably, 3 to 10% by mass relative to the total mass of the adhesive agent layer according to the present invention. If the content of the l-menthyl glyceryl ether is less than the lower limit, the solubilizing power toward diclofenac sodium will be insufficient and the skin permeability of diclofenac tends to lower, while the pain upon peeling the patch tends to increase. On the other hand, if it exceeds the upper limit, diclofenac analog substances are likely to increase, while stickiness or bleed tends to be easily caused.

Also, in the adhesive agent layer according to the present invention, a content of the l-menthyl glyceryl ether is preferably 0.1 to 10 mass parts, and more preferably, 0.4 to 5 mass parts relative to one mass part of the diclofenac sodium. If the content of the l-menthyl glyceryl ether is less than the lower limit, the solubilizing power toward diclofenac sodium will be insufficient and the skin permeability of diclofenac tends to lower, while the pain upon peeling tends to increase. On the other hand, if it exceeds the upper limit, diclofenac analog substances are likely to increase, while stickiness or bleed tends to be easily caused.

In the patch of the present invention, a content of the terpene-based resin is preferably 5 to 40% by mass, and more preferably, 13 to 20% by mass relative to the total mass of the adhesive agent layer according to the present invention. If the content of the terpene-based resin is less than the lower limit, the attachability to the skin tends to lower, while the coating aptness of an adhesive agent layer composition tends to deteriorate. On the other hand, if it exceeds the upper limit, the skin permeability of diclofenac tends to lower, the pain upon peeling tends to increase, and further stickiness or tongue-out tends to be easily caused.

In the adhesive agent layer according to the present invention, a content of the terpene-based resin is preferably 1 to 40 mass parts, and more preferably, 2.7 to 20 mass parts relative to one mass part of the diclofenac sodium. If the content of the terpene-based resin is less than the lower limit, the attachability to the skin tends to lower, while the coating aptness of an adhesive agent layer composition tends to deteriorate. On the other hand, if it exceeds the upper limit, the skin permeability of diclofenac tends to lower, the pain upon peeling tends to increase, and further stickiness or tongue-out tends to be easily caused.

Further, in the adhesive agent layer according the present invention, a total content of the l-menthyl glyceryl ether and the terpene-based resin can be adjusted, as appropriate, depending on the amount of the diclofenac but is preferably 6 to 60% by mass, and more preferably, 18 to 29% by mass relative the total mass of the adhesive agent layer. If the total amount is less than the lower limit, the skin permeability of diclofenac tends to lower and the attachability to the skin tends to lower. On the other hand, if it exceeds the upper limit, the skin permeability of diclofenac tends to lower, while the pain upon peeling tends to increase, and further stickiness or tongue-out tends to be easily caused.

In addition, there is no particular limitation to an adhesive base that the adhesive agent layer according to the present invention comprises and that has pressure-sensitive adhesiveness. The examples include rubber type bases, acrylic type bases, polyurethane type bases, silicone type bases, hydrogels comprised of water-soluble polymers, or mixtures of these. Further, as necessary, a tackifier, a plasticizer, and other additives may be comprised in the adhesive agent layer.

The thus-mentioned rubber type base is a non-aqueous or anhydrous adhesive composition principally consisting of a natural rubber and/or a synthetic rubber. Examples of such synthetic rubber include polyisoprene, polyisobutylene, polybutadiene, styrene-butadiene-styrene block copolymers, styrene-isoprene-styrene block copolymers, styrene-butadiene rubbers, and styrene-isoprene rubbers. Out of these, the styrene-isoprene-styrene block copolymers and/or polyisobutylene is preferable from the standpoints that the cohesion of the patch is enhanced and that the coating aptness of the adhesive agent layer composition is made good.

Also, a total content of the natural rubber and/or the synthetic rubber is preferably 10 to 40% by mass, more preferably 20 to 40% by mass, and most preferably, 25 to 35% by mass relative to the total mass of the adhesive agent layer according the present invention. If the total content is less than the lower limit, exudation (the components of the adhesive agent layer exudating into the support layer), tongue-out, and stickiness are likely to be caused, while the pain upon peeling off a patch tends to increase. On the other hand, if it exceeds the upper limit, the attachability to the skin tends to lower and the coating aptness of an adhesive agent composition tends to deteriorate.

As the-thus mentioned acrylic type base, there are, for example, mentioned adhesive bases obtained by polymerizing or copolymerizing at least one member of butyl acrylate, 2-ethylhexyl acrylate, vinyl acetate, methacrylate, hydroxyethyl acrylate, glycidyl methacrylate, and methoxyethyl acrylate. Specifically, there are mentioned DURO-TAK87-2097, 87-2194, 87-2196, 87-2287, 87-2516, 87-2852 (trade names; manufactured by Henkel Corporation), NISSETU KP-77, and AS-370 (trade names; manufactured by Nippon Carbide Industries Co. Ltd).

Also, a total content of the acrylic type base is preferably 20 to 90% by mass, and more preferably, 40 to 60% by mass relative to the total mass of the adhesive agent layer according to the present invention. If the total content is less than the lower limit or exceeds the upper limit, the adhesiveness, the attachability to the skin for a prolonged time, and the skin permeability of diclofenac tend to lower and the pain upon peeling, skin rash tend to easily increase.

As the thus-mentioned polyurethane type adhesive agent, there can be used an aliphatic type polyurethane adhesive agent or an aromatic type polyurethane adhesive agent, for example. Additionally, as the thus-mentioned silicone type adhesive agent, there can be used those which principally consist of a silicone rubber such as polydimethylsiloxane, polymethylvinylsiloxane, or polymethylphenylsiloxane, for example. Moreover, as the thus-mentioned hydrogel consisting of such a water-soluble polymer, there can be used those which principally consist of gelatin, cargeenan, or hydroxyethyl cellulose.

Also, examples of the tackifier include rosin esters, hydrogenated rosin esters, and maleated rosins. Specifically, there are mentioned ESTER GUM A, AA-G, H, or HP (trade names; Arakawa Chemical Industries, Ltd.), HARIESTER-L, S, or P (trade names; Arakawa Chemical Industries, Ltd.), PINECRYSTAL KE-100 (trade name; Arakawa Chemical Industries, Ltd.), KE-311 (trade name; Arakawa Chemical Industries, Ltd.), HERCOLYN D (trade name; Riken Hercules K. K.), FORAL 85 or 105 (trade names; Riken Hercules K. K.), STEBELITE ESTER 7 or 10 (trade names; Riken Hercules K. K.), and PENTALYN 4820 or 4740 (trade names; Riken Hercules K. K.). In addition, one kind of such tackifiers may be used alone, or two or more kinds may be used in combination.

Further, a total content of the tackifier is preferably 5 to 60% by mass, and more preferably, 10 to 50% by mass relative the total mass of the adhesive agent layer according to the present invention. If the total content is less than the lower limit, the adhesive force and the attachability to the skin for a prolonged time tend to lower. On the other hand, if it exceeds the upper limit, the percutaneous absorbability of diclofenac, and shape-holing property tend to lower, while the pain upon peeling off a patch, skin rash, and stickiness tend to increase.

Also, as the plasticizer, there are mentioned petroleum-based oils (such as paraffin-based processed oils, naphthene-based processed oils or aromatic-based process oils), squalane, squalene, vegetable-based oils (such as almond oil, olive oil, camelia oil, castor oil, tall oil, and peanut oil), dibasic acid esters (such as dibutyl phthalate and dioctyl phthalate), polyglycerol fatty acid esters, and liquid rubbers (such as liquid polybutene and liquid isoprene). One kind of these may be used alone, or two or more kinds may be used in combination. In addition, as the plasticizer, liquid paraffin and a polyglycerol fatty acid ester are preferable from the standpoint that the attachability to the skin tends to improve more.

Furthermore, a total content of the plasticizer is preferably 7 to 70% by mass, more preferably 10 to 60% by mass, and most preferably 30 to 45% by mass relative the total mass of the adhesive agent layer according to the present invention. If the total content is less than the lower limit, the adhesive force, the percutaneous absorbability of diclofenac, and the dispersibility of dichlofenac sodium tend to decrease. On the other hand, if it exceeds the upper limit, the cohesion and the shape-holding property tend to be lowered, and the pain upon peeling off a patch, and stickiness tend to increase.

The adhesive agent layer according to the present invention can be, as necessary, further compounded with an antioxidant (such as ascorbic acid, propyl gallate, butylhydroxyanisole, dibutylhydroxytoluene (BHT), nor-dihydroguaiaretic acid, tocopherol, and tocopherol acetate), an ultraviolet absorber (such as p-aminobenzoic acid, a p-aminobenzoate, amyl p-dimethylaminobenzoate, a salicylate, methyl anthranilate, umbelliferon, esculin, benzyl cinnamate, cinoxate, guaiazulene, urocanic acid, 2-(2-hydroxy-5-methylphenyl)benzotriazole, 4-methoxybenzophenone, 2-hydroxy-4-methoxybenzophenone, octabenzone, dioxybenzone, dihydroxydimethoxybenzophenone, sulisobenzone, benzoresorcinol, octyldimethyl p-aminobenzoate, and ethylhexyl p-methoxycinamate), an antibacterial agent (such as p-hydroxybenzoate, benzoic acid, a benzoate, sorbic acid, a sorbate, dehydroacetate, 4-isopropyl-3-methylphenol, 2-isopropyl-5-methylphenol, hinokitiol, cresol, 2,4,4-trichloro-2'-hydroxydipheneyl ether, 3,4,4'-trichlorocarbanide, and chlorobutanol), a filler (such as aluminum hydroxide, aluminum silicate hydrate, kaolin, titanium oxide, talc, zinc oxide, silica hydrate, magnesium carbonate, calcium hydrogen phosphate, magnesium silicate, diatomaceous earth, silicic acid anhydrate, bentonite, sodium stearate, calcium stearate, potassium stearate, magnesium stearate, and zinc stearate), a refreshing agent (l-menthol), perfume.

Note that the time-dependent stability of diclofenac sodium and/or the skin permeability of diclofenac to be improved by including l-menthyl glyceryl ether and the terpene-based resin tends to easily lower in coexistence with citric acid, a glycol, or a polyalkyleneglycol fatty acid ester, each of which has been conventionally used as a transdermal absorption promoting agent of diclofenac. Accordingly, there is no need for the adhesive agent layer according to the present invention to even comprise citric acid, the glycol, or the polyalkyleneglycol fatty acid ester.

The adhesive agent layer according to the present invention is a layer comprising the above-described components and may be a single layer having a single composition or a multilayer in which a plurality of layers having different compositions are laminated. A thickness of such adhesive agent layer is usually 10 to 500 µm, and preferably 30 to 300 µm, from the standpoints that good coating aptness is easily obtainable, the sense of use is good, further adequate bioavailability of diclofenac is easily obtainable, and the production cost is easily curbed.

Next, a support layer according to the present invention will be described. The support layer to be used in the present invention is to retain the above-described adhesive agent layer. There can be used a film, a sheet, a sheet-like porous product, a sheet-like foaming product, a fabric, a woven fabric, a nonwoven fabric each made of a synthetic resin, including polyethylene, polypropylene, polybutadiene, ethylene-vinyl acetate copolymer, vinyl acetate-vinyl chloride copolymer, polyvinyl chloride, a polyamide, a polyester, nylon, a cellulose derivative, and a polyurethane, as well as paper or a laminate of the foregoing. Out of these, from the standpoint that the attachability of a patch to the skin is more easily secured, one having elasticity such as a woven fabric is preferable; from the standpoint of easy handling of the patch, one having self-supportability (such as a polyester fabric) is preferable. A thickness of such support layer is preferably 200 to 1000 µm.

Thus far, preferred embodiments of the adhesive agent layer and the support layer that the patch of the present invention comprises have been described. The patch of the present invention may further comprise a release liner layer for covering and protecting a surface of the adhesive agent layer until the patch is used. The material of the release liner layer is not particularly limited, but it is preferable to use a sheet material made of a polyester, polypropylene, polyethylene, paper, or a laminate of the foregoing, which has been subjected to releasability treatment (e.g., silicone coating). In addition, a thickness of such release liner layer is preferably 10 to 200 µm.

Furthermore, an area of the patch of the present invention can be usually set within any range of 1 to 1000 cm² and is not particularly limited as long as a skin permeation amount of diclofenac that is therapeutically effective can be attained. However, an area of 50 to 200 cm² is preferable from the standpoint that adhesive force which is sufficient to make attachment easy and which does not allow for being peeled during the attachment is secured.

The patch of the present invention can be produced by employing, as appropriate, a known method for producing a patch, without any particular limitation. For example, various components other than diclofenac sodium, l-menthyl glyceryl ether, a refreshing agent (l-menthol) that compose the adhesive agent layer are first mixed at their predetermined proportions at heating under an inert atmosphere such as nitrogen, and then after diclofenac sodium, l-menthyl glyceryl ether, and the refreshing agent (l-menthol) are added thereto at predetermined proportions, and further stirring is conducted to obtain an uniformly solubilized product. Next, the thus-obtained solubilized product is directly applied onto surface(s) of a support layer (normally onto one surface) at a predetermined thickness following an ordinary method to form an adhesive agent layer. Subsequently, after the surface of the adhesive agent layer that is opposite to the support layer is covered with a release liner layer and is cut into a predetermined shape, whereby the patch of the present invention can be obtained. Alternatively, after the solubilized product is first applied onto one surface of a release liner layer at a predetermined thickness to form an adhesive agent layer, a support layer is compression-transferred onto the surface of the adhesive agent layer that is opposite to the release liner layer and is cut into a predetermined shape, whereby the patch of the present invention may be obtained.

Also, the various components described above and diclofenac sodium may be added to an organic solvent such as hexane, toluene, or ethyl acetate so that their predetermined proportions may be attained, and stirred to obtain a uniform solubilized product (an adhesive agent layer composition). Then, this solubilized product is applied onto one surface of a support layer at a predetermined thickness and dried with a dryer, and the organic solvent is removed by evaporation to form an adhesive agent layer. Subsequently, after the surface of the adhesive agent layer that is opposite to the support layer is covered with a release liner layer and is cut into a predetermined shape, whereby the patch of the present invention can be obtained. Alternatively, the solubilized product is first applied onto one surface of the release liner layer at a predestined thickness, dried with a dryer, and the organic solvent is removed by evaporation to form an adhesive agent layer. Thereafter, the support layer is compression-transferred onto the surface of the adhesive agent layer that is opposite to the release liner layer and is cut into a predetermined shape, whereby the patch of the present invention may be obtained.

### [Examples]

Hereinafter, the present invention will be described more specifically based on Examples and Comparative Examples.

First, in order to obtain suitable solubilizers for diclofenac sodium, 5 parts by mass of candidate solubilizers relative to 1 part by mass of diclofenac sodium were added to sample tubes and heated at 100 °C for 2 hours. Next, the solubilities of diclofenac sodium immediately after the heating were evaluated based on four levels of indices as described below. Also, after the heating, standing was allowed at room temperature (25 °C) for a day. Further, the solubilities of diclofenac sodium after the lapse of one day were evaluated based on the indices as described below. The obtained results are shown in Table 1.
A: Completely dissolved
B: Some undissolved remnants observed
C: Hard to be dissolved and a lot of undissolved remnants observed
D: Scarcely dissolved

**[Table 1]**

| Additive | Solubilizing power Immediately after heating | After lapse of one day |
|---|---|---|
| 1-Menthyl glyceryl ether | A | A |
| 1-Menthol | D | D |
| Crotamiton | D | D |
| Triacetin | D | D |
| Pyrrothiodecane | D | B |
| Propylene glycol | A | A |
| N-Methyl-2-pyrrolidone | A | A |
| Concentrated glycerin | A | A |
| Polyoxyethylene(2) lauryl ether | A | A |
| Polyoxyethylene(9) lauryl ether | A | A |
| Polyoxyethylene(7) oleyl ether | B | A |
| Polyethylene glycol monooleate (6E.O.) | B | A |
| Polyethylene glycol monolaurate (10E.O.) | C | Solidified |
| Polyethylene glycol monostearate (10E.O.) | B | Solidified |
| Polyethylene glycol monostearate (2E.O.) | B | Solidified |
| Polyethylene glycol monostearate (4E.O.) | B | Solidified |

As is evident from the results shown in Table 1, solubilizers such as l-menthyl glyceryl ether are suitable as solubilizers of diclofenac sodium. However, it became clear that l-menthol, which had the same partial skeleton as that of l-menthyl glyceryl ether, was insufficient as the solubilizer of diclofenac sodium.

### (Comparative Examples 1-7)

Next, the components listed in Table 2 were weighted out so that they were to be respectively predetermined mass percentages. Subsequently, styrene-isobutylene-styrene block copolymer, polyisobutylene, an alicyclic saturated hydrocarbon resin (ARKON P-100), liquid paraffin, and other components were charged into a mixer heated at 200 °C and heat-melted while maintaining such a number of revolution that the content was sufficiently stirred under a circulation of nitrogen. Subsequently, the same amount of the alicyclic saturated hydrocarbon resin (ARKON P-100) as that added before was charged and heat-melting was further continued. Then, at the point of being heat-melted, diclofenac sodium, l-menthol, citric acid, and various solubilizers were charged while maintaining the temperature of the content at 110 to 130 °C, kneaded and blended. At the point the content turned to a uniform state, it was applied onto one surface of a release liner layer (material: PET, surface treatment: silicone treatment) while being maintained at 100 to 120 °C. Next, the surface that was opposite to the release liner layer was covered with a support layer (material: polyester) and pressure-adhered, whereby a patch was obtained. Further, the obtained patches were evaluated for the stabilities of diclofenac sodium according the method and index as described below. The obtained results are shown in Table 2.

### (Stability Test)

The obtained patch was stored at 60 °C, and the content of diclofenac sodium after 2 weeks was measured by high performance liquid chromatography (HPLC). Further, the content (% by mass) of diclofenac sodium in the adhesive agent layer of the patch after storage at 60 °C for 2 weeks was calculated relative to the content of diclofenac sodium in the adhesive agent layer of the patch before the storage at 60 °C being made 100. Note that it was empirically ascertained that the storage at 60 °C for 2 weeks was equivalent to storage at 40 °C for 3 months or storage at 25 °C for 18 months. When the content of diclofenac sodium after the storage at 60 °C for 2 weeks is a value of 97.5% by mass or more as compared with that before the storage, it will be presumed that diclofenac sodium can be stably comprised and its efficacy can be guaranteed at 25 °C for 3 years. Therefore, the stability of diclofenac sodium was evaluated on the basis of the value of "97.5% by mass."

**[Table 2]**

| | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| | Diclofenac sodium | 1 | 1 | 1 | 1 | 1 | 1 | 1 |
| | 1-Menthol | 1.5 | 1.5 | 1.5 | 1.5 | 1.5 | 1.5 | 1.5 |
| | Citric acid | 0.4 | 0.4 | 0.4 | 0.4 | 0.4 | 0.4 | 0.4 |
| | Styrene-isobutylene-styrene block copolymer | 19.69 | 19.69 | 19.69 | 19.69 | 19.69 | 19.69 | 19.69 |
| | Polyisobutylene | 12.6 | 12.6 | 14.5 | 12.6 | 12.6 | 12.6 | 12.6 |
| | Alicyclic saturated hydrocarbon resin | 13.13 | 13.13 | 13.13 | 13.13 | 13.13 | 13.13 | 13.13 |
| | Liquid paraffin | 43.58 | 43.58 | 43.18 | 43.58 | 43.58 | 43.58 | 43.58 |
| | Other components | 4.1 | 4.1 | 2.6 | 4.1 | 4.1 | 4.1 | 4.1 |
| solubilizer | Polyoxyethylene(2) lauryl ether | 4 | - | - | - | - | - | - |
| | Polyoxyethylene(9) lauryl ether | - | 4 | - | - | - | - | - |
| | N-Methyl-2-pyrrolidone | - | - | 4 | - | - | - | - |
| | Polyoxyethylene(7) oleyl ether | - | - | - | 4 | - | - | - |
| | Polyethylene glycol monooleate (6E.O.) | - | - | - | - | 4 | - | - |
| | Polyethylene glycol monolaurate (10E.O.) | - | - | - | - | - | 4 | - |
| | Polyethylene glycol monostearate (10E.O.) | - | - | - | - | - | - | 4 |
| | Stability of diclofenac sodium (%) | 76.4 | 85.4 | 93.9 | 81.9 | 90.1 | 80.2 | 81.5 |

As is evident from the results shown in Table 2, any of the solubilizers when used is insufficient in the stability of diclofenac sodium (a content of diclofenac sodium being less than 97.5% by mass) with respect to the adhesive agent layers comprising citric acid.

### (Reference Example 1 and Comparative Examples 8-17)

Next, the components listed in Table 3 were weighted out so that they were to be respectively predetermined mass percentages. Subsequently, styrene-isobutylene-styrene block copolymer, polyisobutylene, an alicyclic saturated hydrocarbon resin (ARKON P-100), liquid paraffin, and other components were charged into a mixer heated at 200 °C and heat-melted while maintaining such a number of revolution that the content was sufficiently stirred under a circulation of nitrogen. Subsequently, the same amount of the alicyclic saturated hydrocarbon resin (ARKON P-100) as that added before was charged and heat-melting was further continued. Then, at the point of being heat-melted, diclofenac sodium, l-menthol, citric acid, and various solubilizers were charged while maintaining the temperature of the content at 110 to 130 °C, kneaded and blended. At the point the content turned to a uniform state, it was applied onto one surface of a release liner layer (material: PET, surface treatment: silicone treatment) while being maintained at 100 to 120 °C. Next, the surface that was opposite to the release liner layer was covered with a support layer (material: polyester) and pressure-adhered, whereby a patch was obtained. Further, the obtained patches were evaluated for the stabilities of diclofenac sodium according the method and index as described below. In addition, the skin permeabilities of diclofenac were evaluated according to the method described below. The obtained results are shown in Table 3.

### (Skin Permeation Test)

Skin on the back of a hairless mouse was peeled off and was mounted to a Franz-type flow-through cell in which hot water at 32 °C was circulated through its outer peripheral part, so that the dermis side of the skin was conformed to the receptor chamber side. Next, a patch which had been cut into a size of 4.5 cm² and from which a release liner layer had been removed was attached on the corneum side of this skin. A phosphate buffer solution (pH 7.4) was caused to flow in the receptor chamber of the flow-through cell at a flow rate of 5 mL/hr, and sample liquids were collected from the receptor chamber every 4 hours for 24 hours' portion from the start of measurement. Each of the collected sample liquids was measured for the concentration of the drug (diclofenac) by high performance liquid chromatography (HPLC). With respect to a 1% diclofenac sodium-containing patch that was commercially available, the drug concentration (diclofenac) was measured by following the same method. Further, based on the measurement value obtained, the value which was (measurement in each patch)/(measurement of the commercially available 1% diclofenac-containing patch) X 100 was calculated, and the skin permeability of diclofenac in each patch was evaluated.

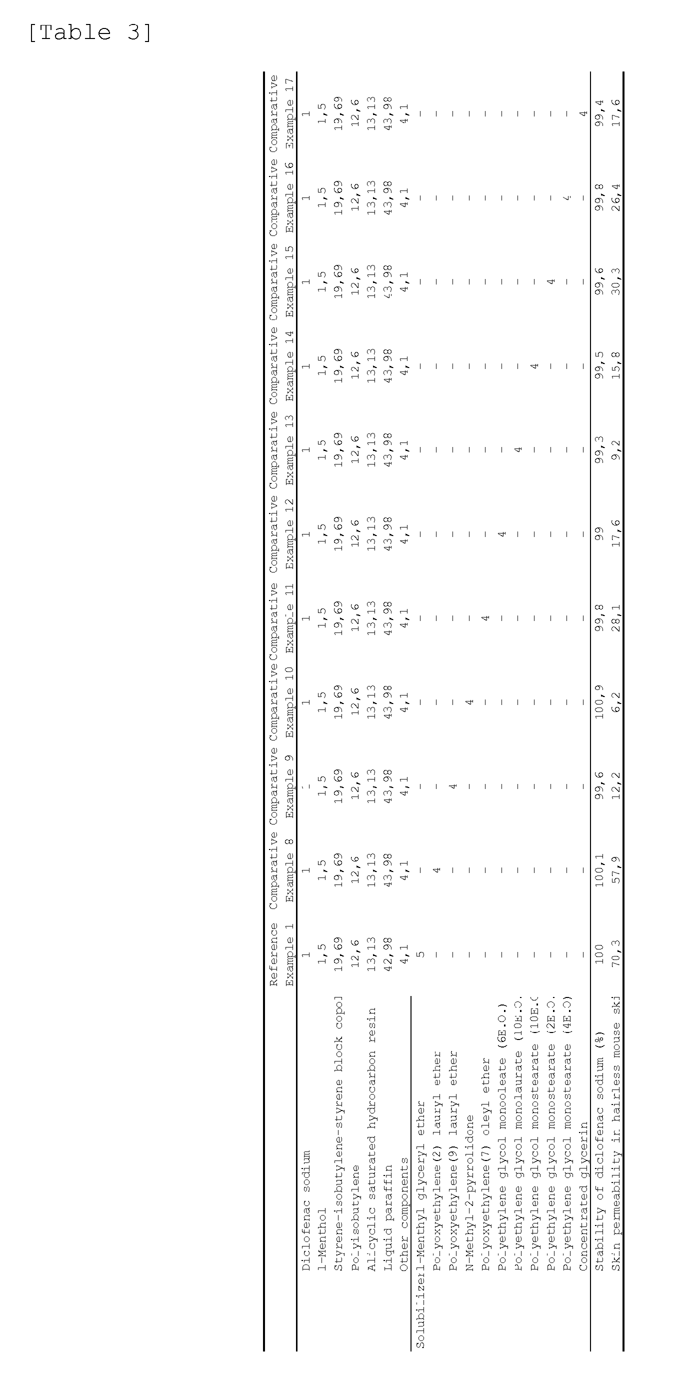

As is evident from the results shown in Table 3, any of the solubilizers when used was sufficient in the stability of diclofenac sodium (a content of diclofenac sodium being 97.5% by mass or more) with respect to the adhesive agent layers comprising no citric acid. When 1-menthyl glyceryl ether was used as the solubilizer (Reference Example 1), the skin permeability of dichlofenac was also excellent. On the other hand, when the solubilizers according to the present invention were not comprised (Comparative Examples 8-17), the skin permeabilities of diclofenac were inferior.

### (Examples 2, and 4-8, Reference Examples 1 and 3 and Comparative Examples 18-21)

Next, the components listed in Table 4 were weighted out so that they were to be respectively predetermined mass percentages. Subsequently, styrene-isobutylene-styrene block copolymer, polyisobutylene, an alicyclic saturated hydrocarbon resin (ARKON P-100), a pinene polymer (YS resin) or a hydrogenated rosin ester (KE-311), liquid paraffin, and other components were charged into a mixer heated at 200 °C and heat-melted while maintaining such a number of revolution that the content was sufficiently stirred under a circulation of nitrogen. Subsequently, the same amount of the alicyclic saturated hydrocarbon resin, the pinene polymer or the hydrogenated rosin ester as that added before was charged and heat-melting was further continued. Then, at the point of being heat-melted, diclofenac sodium, l-menthol, and the solubilizer being 1-menthyl glyceryl ether, propylene glycol, or polyethylene glycol monostearate (2E.O.) were charged while maintaining the temperature of the content at 110 to 130 °C and mixed. At the point the content turned to a uniform state, it was applied onto one surface of a release liner layer (material: PET, surface treatment: silicone treatment) while being maintained at 100 to 120 °C. Next, the surface that was opposite to the release liner layer was covered with a support layer (material: polyester) and pressure-adhered, whereby a patch was obtained. Further, the obtained patches (Examples 2 and 4, Refernce Examples 1 and 3, and Comparative Examples 18, 20, 21) were evaluated for the stabilities of diclofenac sodium according the method and index as described below. In addition, the obtained patches (Examples 5-8) were evaluated for the stabilities of diclofenac sodium when the storage conditions in the stability test described before were changed from "at 60 °C for 2 weeks" to "at 40 °C for 6 months." Note that the storage at 60 °C for 2 weeks was equivalent to the storage at 40 °C for 3 months as previously mentioned. Therefore, Examples 5-8 turned out to having been subjected to the stability test for a double period as compared with Examples 2 and 4 and Reference Examples 1 and 3. Further, the obtained patches (Example 2, Reference Examples 1 and 3, Examples 5-8 and Comparative Examples 18-21) were evaluated for the skin permeabilities of diclofenac according to the method described below. Moreover, the obtained patches (Example 2 and Reference Example 3 as well as Comparative Examples 20 and 21) were evaluated for irritation to the skin. The obtained results are shown in Tables 4 and 5.

### (Evaluation Test on Irritation to Skin)

Each patch which had been cut into a circular size with a diameter of 1.5 cm and from which a release liner layer had been removed was attached to the back parts of 30 healthy male adults. Then, after 48 hours, each patch was peeled off, and the skin irritation index (SI value) after peeling was determined. Note that the skin irritation index was determined according to the Sugai method (Skin, Volume 27, No. 4, August of Showa 60, vid.).

**[Table 4]**

| | Reference Example 1 | Example 2 | Reference Example 3 | Example 4 | Comparative Example 18 | Comparative Example 19 | Comparative Example 20 | Comparative Example 21 |
|---|---|---|---|---|---|---|---|---|
| Diclofenac sodium | 1 | 1 | 1 | 1 | 1 | 1 | 1 | 1 |
| 1-Menthol | 1,5 | 3,5 | 3 | 3 | 3 | 1,5 | 3 | 3 |
| Ammonium chloride | - | - | - | - | - | 0,25 | - | - |
| Styrene-isobutylene-styrene block copolymer | 19,69 | 20 | 19,69 | 20 | 22 | 17,95 | 25 | 25 |
| Polyisobutylene | 12,6 | 7 | 12,6 | 7 | 9,5 | 8,71 | 9,5 | 9,5 |
| Terpene resin (YS RESIN) | - | 20 | - | 26 | - | - | - | - |
| Alicyclic saturated hydrocarbon resin (ARKON) | 13,13 | - | 16 | - | - | - | - | - |
| Hydrogenated rosin ester (KE-311) | - | - | - | - | 16 | 21,65 | 13 | 13 |
| Liquid paraffin | 42,98 | 42,4 | 39,6 | 36,9 | 41,05 | 39,72 | 41,05 | 41,05 |
| Other components | 4,1 | 3,1 | 3,1 | 3,1 | 2,45 | 9,22 | 2,45 | 2,45 |
| Solubilizer (1-menthyl glyceryl ether) | 5 | 3 | 5 | 3 | 5 | - | 3 | 3 |
| Solubilizer (propylene glycol) | - | - | - | - | - | - | 2 | - |
| Solubilizer (polyethylene glycol monostearate ) | - | - | - | - | - | - | - | 2 |
| Stability of diclofenac sodium (%) | 100 | 99,6 | 100,1 | 99,8 | 94,6 | - | 94,9 | 93,1 |
| [test conditions: storage at 60 °C for 2 weeks] | | | | | | | | |
| | | | | | | | | |
| Skin permeability in hairless mouse skin | 70,3 | 97,5 | 120,9 | - | 60,9 | 96,8 | 78,4 | 73,6 |
| Skin irritation (SI value) | - | 20 | 46,7 | - | - | - | 35 | 41,7 |

**[Table 5]**

| | Example 5 | Example 6 | Example 7 | Example 8 |
|---|---|---|---|---|
| Diclofenac sodium | 5 | 7 | 5 | 7 |
| 1-Menthol | 3.5 | 3.5 | 3.5 | 3.5 |
| Ammonium chloride | - | - | - | - |
| Styrene-isobutylene-styrene block copolymer | 19 | 18.5 | 19 | 18.5 |
| Polyisobutylene | 6.86 | 6.76 | 6.86 | 6.76 |
| Terpene resin (YS RESIN) | 19 | 19 | 19 | 19 |
| Alicyclic saturated hydrocarbon resin (ARKON) | - | - | - | - |
| Hydrogenated rosin ester (KE-311) | - | - | - | - |
| Liquid paraffin | 40.54 | 39.14 | 33.54 | 32.14 |
| Other components | 3.1 | 3.1 | 3.1 | 3.1 |
| Solubilizer (1-menthyl glyceryl ether) | 3 | 3 | 10 | 10 |
| Solubilizer (propylene glycol) | - | - | - | - |
| Solubilizer (polyethylene glycol monostearate (2E.O.) | - | - | - | - |
| Stability of diclofenac sodium (%) | 100 | 100 | 99.5 | 99.4 |
| [test conditions: storage at 40 °C for 6 months] | | | | |
| Skin permeability in hairless mouse skin | 117.8 | 117.7 | 250 | 139.8 |
| Skin irritation (SI value) | - | - | - | - |

As is evident from the results shown in Tables 4 and 5, the patches of the present invention, i.e., patches of which the adhesive layers comprise l-menthyl glyceryl ether, the terpene-based resin (Examples 2, and 4-8) were sufficient in the stabilities of diclofenac sodium (contents of diclofenac sodium being 97.5% by mass or more) and were also excellent from the standpoint of the percutaneous permeability of diclofenac. Furthermore, it became clear that among the patches of the present invention, the patch (Example 2) comprising the terpene-based resin in its adhesive agent layer had less skin irritability as compared with the patch (Reference Example 3) comprising the petroleum-based resin in its adhesive agent layer.

Meanwhile, the patches of which the adhesive agent layers do not comprise the terpene-based resin (Comparative Examples 18, 20 and 21) were insufficient in the stabilities of diclofenac sodium (contents of diclofenac sodium being less than 97.5% by mass)

### [Industrial Applicability]

As described above, according to the present invention, it is possible to provide a patch which can stably comprise diclofenac sodium and which at the same time is excellent in the skin permeability of diclofenac. Accordingly, the patch of the present invention is useful as an analgesic anti-inflammatory patch.

## Claims

1. A transdermal absorption promoting agent for promoting percutaneous absorption of diclofenac sodium, comprising l-menthyl glyceryl ether and a terpene-based resin, wherein the terpene-based resin is a pinene polymer.

2. The transdermal absorption promoting agent according to claim 1, wherein a mass ratio between the l-menthyl glyceryl ether and the terpene-based resin (mass of menthyl glyceryl ether: mass of terpene-based resin) is 1:0.1 to 1:20.

3. The transdermal absorption promoting agent according to claim 1 or 2, further comprising none of citric acid, a glycol, and a polyalkyleneglycol fatty acid ester.

4. The transdermal absorption promoting agent according to claim 1, wherein a mass ratio between the l-menthyl glyceryl ether and the terpene-based resin (mass of menthyl glyceryl ether: mass of terpene-based resin) is 1:0.1 to 1:20 and further comprising none of citric acid, a glycol, and a polyalkyleneglycol fatty acid ester.

5. A patch comprising a support layer and an adhesive agent layer, wherein the adhesive agent layer comprises diclofenac sodium and further comprises the transdermal absorption promoting agent of any one of claims 1-4 to promote percutaneous absorption of diclofenac sodium.

6. The patch according to claim 5, wherein the adhesive agent layer comprises 0.1 to 10 mass parts of the l-menthyl glyceryl ether and 1 to 40 mass parts of the terpene-based resin relative to 1 mass part of the diclofenac sodium.

7. The patch according to claim 5 or 6, comprising none of citric acid, a glycol and a polyalkyleneglycol fatty acid ester in the adhesive agent layer.

8. The patch according to claim 5, wherein the adhesive agent layer comprises 0.1 to 10 mass parts of the l-menthyl glyceryl ether and 1 to 40 mass parts of the terpene-based resin relative to 1 mass part of the diclofenac sodium,
the adhesive agent layer comprises the l-menthyl glyceryl ether and the terpene-based resin in a total content of 18 to 29% by mass relative to a total mass of the adhesive agent layer, and
the adhesive agent layer comprises none of citric acid, a glycol and a polyalkyleneglycol fatty acid ester in the adhesive agent layer.

## Patentansprüche

1. Transdermales absorptionsförderndes Mittel zur Förderung der perkutanen Absorption von Diclofenac-Natrium, umfassend L-Menthylglycerylether und ein Harz auf Terpenbasis, wobei das Harz auf Terpenbasis ein Pinenpolymer ist.

2. Transdermales absorptionsförderndes Mittel nach Anspruch 1, wobei das Massenverhältnis zwischen dem L-Menthylglycerylether und dem Harz auf Terpenbasis (Masse des Menthylglycerylethers : Masse des Harzes auf Terpenbasis) 1 : 0,1 bis 1 : 20 beträgt.

3. Transdermales absorptionsförderndes Mittel nach Anspruch 1 oder 2, das ferner keine Zitronensäure, kein Glycol und keinen Polyalkylenglycolfettsäureester umfasst.

4. Transdermales absorptionsförderndes Mittel nach Anspruch 1, wobei das Massenverhältnis zwischen dem L-Menthylglycerylether und dem Harz auf Terpenbasis (Masse des Menthylglycerylethers : Masse des Harzes auf Terpenbasis) 1 : 0,1 bis 1 : 20 beträgt und das ferner keine Zitronensäure, kein Glycol und keinen Polyalkylenglycolfettsäureester umfasst.

5. Pflaster, umfassend eine Trägerschicht und eine Klebemittelschicht, wobei die Klebemittelschicht Diclofenac-Natrium umfasst und ferner das transdermale absorptionsfördernde Mittel nach einem der Ansprüche 1-4 umfasst, um die perkutane Absorption von Diclofenac-Natrium zu fördern.

6. Pflaster nach Anspruch 5, wobei die Klebemittelschicht 0,1 bis 10 Massenteile des L-Menthylglycerylethers und 1 bis 40 Massenteile des Harzes auf Terpenbasis, bezogen auf 1 Massenteil des Diclofenac-Natriums, umfasst.

7. Pflaster nach Anspruch 5 oder 6, das keine Zitronensäure, kein Glycol und keinen Polyalkylenglycolfettsäureester in der Klebemittelschicht umfasst.

8. Pflaster nach Anspruch 5, wobei die Klebemittelschicht 0,1 bis 10 Massenteile des L-Menthylglycerylethers und 1 bis 40 Massenteile des Harzes auf Terpenbasis, bezogen auf 1 Massenteil des Diclofenac-Natriums, umfasst,
die Klebemittelschicht den L-Menthylglycerylether und das Harz auf Terpenbasis in einem Gesamtgehalt von 18 bis 29 Massen-%, bezogen auf die Gesamtmasse der Klebemittelschicht, umfasst, und
die Klebemittelschicht keine Zitronensäure, kein Glycol und keinen Polyalkylenglycolfettsäureester in der Klebemittelschicht umfasst.

## Revendications

1. Agent favorisant l'absorption transdermique pour favoriser l'absorption percutanée du diclofénac sodique, comprenant de l'éther de I-menthyl glycéryle et une résine à base de terpène, la résine à base de terpène étant un polymère pinène.

2. Agent favorisant l'absorption transdermique selon la revendication 1, un rapport en masse entre l'éther de I-menthyl glycéryle et la résine à base de terpène (masse de l'éther de menthyl glycéryle:masse de la résine à base de terpène) étant de 1:0,1 à 1:20.

3. Agent favorisant l'absorption transdermique selon la revendication 1 ou 2, ne comprenant en outre aucun de l'acide citrique, d'un glycol, et d'un ester d'acide gras de poly(alcylène glycol).

4. Agent favorisant l'absorption transdermique selon la revendication 1, un rapport en masse entre l'éther de I-menthyl glycéryle et la résine à base de terpène (masse de l'éther de menthyl glycéryle:masse de la résine à base de terpène) étant de 1:0,1 à 1:20 et ne comprenant en outre aucun de l'acide citrique, d'un glycol, et d'un ester d'acide gras de poly(alcylène glycol).

5. Timbre comprenant une couche formant support et une couche d'agent adhésif, la couche d'agent adhésif comprenant du diclofénac sodique et comprenant en outre l'agent favorisant l'absorption transdermique selon l'une quelconque des revendications 1 à 4 pour favoriser l'absorption percutanée du diclofénac sodique.

6. Timbre selon la revendication 5, la couche d'agent adhésif comprenant de 0,1 à 10 parties en masse de l'éther de I-menthyl glycéryle et de 1 à 40 parties en masse de la résine à base de terpène par rapport à 1 partie en masse du diclofénac sodique.

7. Timbre selon la revendication 5 ou 6, ne comprenant aucun de l'acide citrique, d'un glycol et d'un ester d'acide gras de poly(alcylène glycol) dans la couche d'agent adhésif.

8. Timbre selon la revendication 5, la couche d'agent adhésif comprenant de 0,1 à 10 parties en masse de l'éther de I-menthyl glycéryle et de 1 à 40 parties en masse de la résine à base de terpène par rapport à 1 partie en masse du diclofénac sodique,
la couche d'agent adhésif comprenant l'éther de I-menthyl glycéryle et la résine à base de terpène en une teneur totale de 18 à 29 % en masse par rapport à une masse totale de la couche d'agent adhésif, et
la couche d'agent adhésif ne comprenant aucun de l'acide citrique, d'un glycol et d'un ester d'acide gras de poly(alcylène glycol) dans la couche d'agent adhésif.
